(19) Europäisches Patentamt
European Patent Office
Office européen des brevets

(11) **EP 2 600 284 A1**

(12) **DEMANDE DE BREVET EUROPEEN**

(43) Date de publication:
**05.06.2013 Bulletin 2013/23**

(51) Int Cl.:
**G06K 9/00** *(2006.01)*  **G06K 9/62** *(2006.01)*

(21) Numéro de dépôt: **11306609.6**

(22) Date de dépôt: **02.12.2011**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(71) Demandeur: **bioMérieux, Inc.**
**Durham, NC 27712 (US)**

(72) Inventeurs:
• **Strubel, Grégory**
**04100 MANOSQUE (FR)**
• **Arsac, Maud**
**42400 SAINT CHAMOND (FR)**

• **Desseree, Denis**
**01120 MONTLUEL (FR)**
• **Cotte-Pattat, Pierre-Jean**
**01150 LAGNIEU (FR)**
• **Mahe, Pierre**
**38000 GRENOBLE (FR)**

(74) Mandataire: **Le Mauff, Frederic Francis Joseph et al**
**Cabinet Laurent et Charras**
**"Le Contemporain"**
**50, chemin de la Bruyère**
**69574 Dardilly Cedex (FR)**

(54) **Procédé d'identification de microorganismes par spectrométrie de masse et normalisation de scores**

(57) Une indentification par spectrométrie de masse d'un microorganisme parmi des microorganismes de référence représentés par des ensembles de données de référence, comporte :
■ la détermination d'un ensemble de données du microorganisme en fonction d'un spectre ;
■ pour chaque microorganisme de référence, le calcul d'une distance entre les ensembles déterminé et de référence ; et
■ le calcul d'une probabilité $f(m)$ selon la relation :

$$f(m) = \frac{pN(m|\mu,\sigma)}{pN(m|\mu,\sigma) + (1-p)N(m|\overline{\mu},\overline{\sigma})}$$

où :
■ $m$ est la distance calculée pour le microorganisme de référence ;
■ $N(m|\mu,\sigma)$ est la valeur, pour $m$, d'une variable aléatoire modélisant la distance entre un microorganisme de référence à identifier et le microorganisme de référence, lorsque le microorganisme est celui de référence ;
■ $N(m|\overline{\mu},\overline{\sigma})$ est la valeur, pour $m$, d'une variable aléatoire modélisant la distance entre un microorganisme à identifier et le microorganisme de référence, lorsque le microorganisme n'est pas celui de référence ; et
■ $p$ est un scalaire compris entre 0 et 1.

EP 2 600 284 A1

**Fig. 6**

**Description**

**DOMAINE DE L'INVENTION**

La présente invention concerne l'identification de microorganismes, notamment de bactéries, au moyen de la spectrométrie de masse.

**ETAT DE LA TECHNIQUE**

Il est connu d'utiliser la spectrométrie de masse pour identifier des micro-organismes, et plus particulièrement des bactéries. Un échantillon du micro-organisme est préparé puis un spectre de masse de l'échantillon est acquis et pré-traité, notamment pour éliminer la ligne de base (communément appelé « baseline ») et pour éliminer le bruit. Les pics du spectre pré-traité sont alors détectés et la liste de pics ainsi obtenue est « analysée » et « comparée » à l'aide d'outil de classification à des données d'une base de connaissances construites à partir de listes de pics chacune associés à un microorganisme ou à un groupe de micro-organismes (souche, classe, famille, etc...) identifié.

Parmi les outils de classifications, on connaît les classifications SVM (pour « *Support Vector Machine* ») du type « un contre tous » (ci-après classification « SVM-UCT »). Une classification SVM du type « un contre tous » consiste à déterminer, pour chaque classe d'objets d'un ensemble de classes, une frontière orientée qui sépare cette classe des autres classes de l'ensemble. On obtient donc autant de classificateurs « un contre tous » qu'il existe de classes dans l'ensemble. L'identification d'un objet inconnu consiste alors à interroger chacun des classificateurs en calculant la distance algébrique entre l'objet inconnu et la frontière associée au classificateur. Usuellement, l'objet inconnu est déterminé comme appartenant à la classe associé à la plus grande distance calculée.

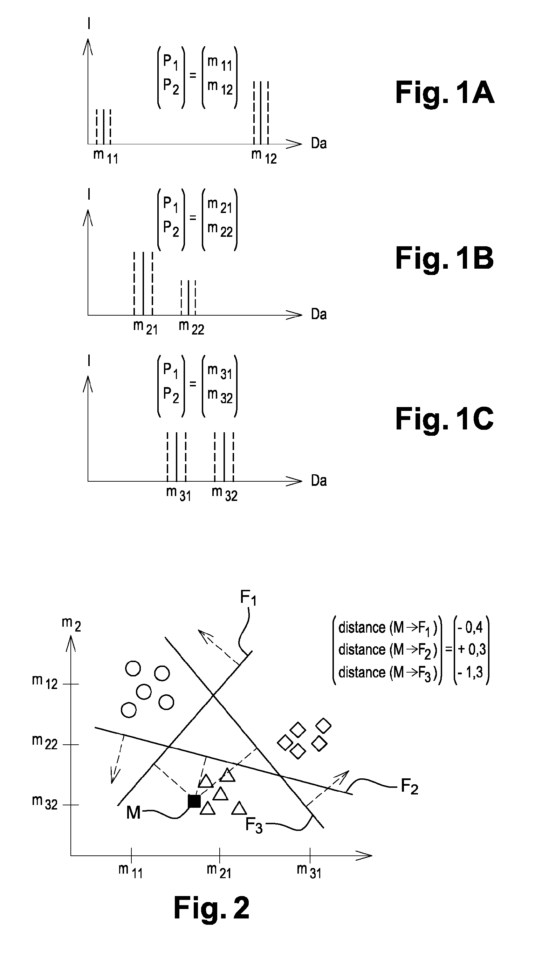

Ce principe est sommairement illustré aux figures 1 et 2 qui illustrent le cas très simple de 3 microorganismes identifiables au moyen de seulement deux pics dans un spectre de masse, par exemple les deux pics de plus forte intensité dans le spectre de masse des 3 microorganismes. Le premier microorganisme est caractérisé par un premier pic localisé sur une valeur $m_{11}$ et un second pic localisé sur une valeur $m_{12}$ (figure 1A), le deuxième microorganisme est caractérisé par un premier pic localisé sur une valeur $m_{21}$ et un second pic localisé sur une valeur $m_{22}$ (figure 1B), et le troisième microorganisme est caractérisé par un premier pic localisé sur une valeur $m_{31}$ et un second pic localisé sur une valeur $m_{32}$ (figure 1C).

La classification de type SVM-UTC consiste dans un premier temps à acquérir un ensemble de spectres de masse d'apprentissage de chacun des microorganismes et de déterminer la localisation des deux pics en cause dans chaque spectre acquis de manière à former un ensemble de vecteurs $\begin{pmatrix} p_1 \\ p_2 \end{pmatrix}$ d'apprentissage, $p_1$ étant la position mesurée du premier pic, et p2 étant la position mesurée du second pic. En raison de l'incertitude sur la mesure, on observe une dispersion des valeurs des vecteurs. Dans une seconde étape, il est calculé pour chaque microorganisme une frontière séparant l'ensemble des vecteurs $\begin{pmatrix} p_1 \\ p_2 \end{pmatrix}$ associés au microorganisme, des vecteurs $\begin{pmatrix} p_1 \\ p_2 \end{pmatrix}$ associés aux deux autres microorganismes. Trois frontières $F_1$, $F_2$ et $F_3$ sont ainsi obtenue, telles que représentées à la figure 2, et sont munies d'une orientation, par exemple celle représentée par les flèches en traits pointillés.

L'identification d'un microorganisme inconnu consiste alors à acquérir un ou plusieurs spectres de masse de microorganisme, d'en déduire un vecteur *M* de pics $\begin{pmatrix} p_1 \\ p_2 \end{pmatrix}$ mesuré, et de calculer la distance algébrique, également nommée « marge », de ce vecteur *M* à chacune des frontières orientées $F_1$, $F_2$ et $F_3$. Il est ainsi obtenu un vecteur de distances algébriques, par exemple égal à $\begin{pmatrix} -0,4 \\ +0,3 \\ -1,3 \end{pmatrix}$. Dans le cas très simple illustré, on pourrait donc en déduire que le microorganisme inconnu est le deuxième microorganisme.

Bien entendu, le cas illustré ici est extrêmement simple. Dans la réalité, il s'agit d'identifier un microorganisme parmi des centaines de microorganismes avec un nombre de pics retenus pour l'identification pouvant dépasser sensiblement 1000 pics. En outre, le cas illustré est également simple puisque les microorganismes sont très éloignés les uns des autres et que la mesure a été réalisée avec suffisamment de précision pour pouvoir déduire des distances une information pertinente.

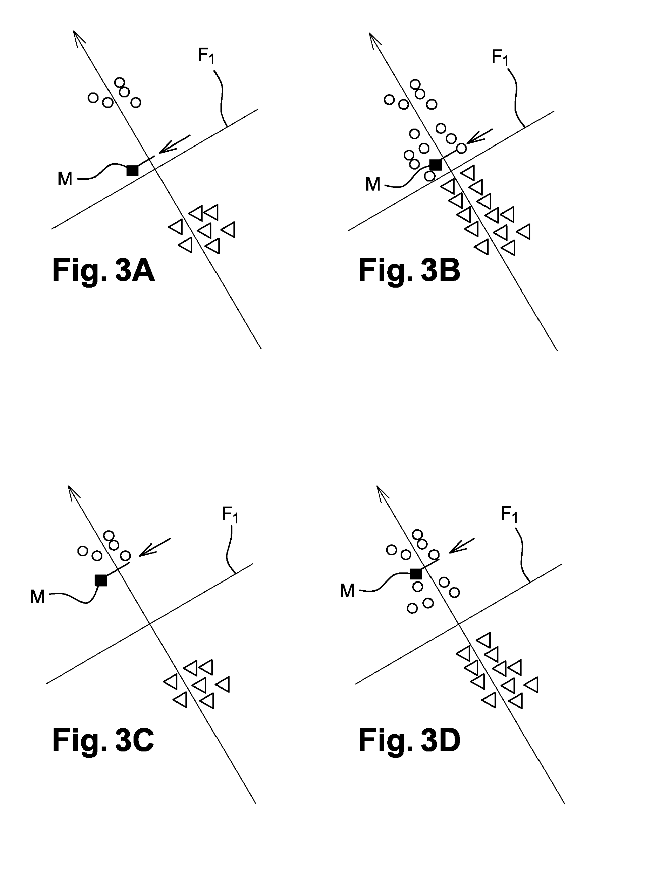

Dans les cas réels, il est difficile, voire impossible, de déduire directement une information pertinente sur les distances calculées aux frontières. En effet, une valeur de distance peut correspondre à des situations très différentes. Les figures 3A à 3D illustrent de manière simple ce principe. Sur ces figures sont représentés la frontière $F_1$ séparant

un ensemble de vecteurs de pics $\begin{pmatrix} m_1 \\ m_2 \end{pmatrix}$ d'apprentissage associés à un premier microorganisme, représentés par des rond, des autres vecteurs de pics $\begin{pmatrix} m_1 \\ m_2 \end{pmatrix}$ d'apprentissage associés aux autres microorganismes, représentés par des triangles. Des vecteurs de pics mesurés M d'un microorganisme inconnu à identifier sont représentés par des carrées.

Dans le cas illustré à la figure 3A, la distance du vecteur mesuré $M$ à la frontière $F_1$ est positive. Toutefois le vecteur $M$ est si éloigné de l'ensemble de vecteur d'apprentissage du premier microorganisme qu'il ne peut pas être déduit avec certitude que le microorganisme inconnu est bien le premier microorganisme. Dans le cas illustré à la figure 3B, le vecteur mesuré $M$ est à présent proche de l'ensemble de vecteur d'apprentissage mais également très proche des autres ensembles de vecteur d'apprentissage. Dans ce cas également, il est difficile de déduire que le microorganisme inconnu est le premier microorganisme. Dans le cas illustré à la figure 3C, le vecteur mesuré $M$ est éloigné de la frontière $F_1$ et est proche de l'ensemble de vecteurs d'apprentissage, tout en étant en bordure de cet ensemble. Bien que ce cas soit plus favorable que les cas précédents, il existe encore une incertitude sur l'appartenance du microorganisme à identifier. Une étude de la précision de la mesure est notamment nécessaire. Enfin, le cas illustré à la figure 3D est le cas typique, et rare, où le vecteur mesuré est à la fois éloigné de la frontière et est localisé parmi l'ensemble des vecteurs d'apprentissage. La distance mesurée est alors une valeur caractéristique du premier microorganisme, dans laquelle on peut avoir confiance.

Comme il est possible de le constater, les distances calculées ne sont que partiellement pertinentes. Par exemple dans un premier cas, une distance égale à 0,4 est extrêmement pertinente alors que dans un autre cas, il est impossible d'en déduire quoique ce soit. Il est donc nécessaire de procéder à une analyse de ces distances pour en déduire le type de microorganisme inconnu, ainsi que le degré de confiance que l'on peut accorder à cette identification. Cette étape d'analyse supplémentaire est classiquement réalisée par un opérateur, biologiste ou médecin, qui détermine à l'aide de son savoir faire quelle conclusion il est possible de tirer des distances calculées par l'outil de classification.

Il a été décrit une classification vectorielle, de type SVM, qui calcule une distance algébrique entre deux objets d'un espace vectoriel, à savoir un vecteur correspondant au microorganisme à identifier, et un hyperplan correspondant à une frontière partitionnant l'espace, dans l'exemple illustré $\Re^2$, en deux sous espace. Le type de problème exposé en relation avec ce type de classification se retrouve également dans les autres types de classification dès lors que celles-ci produisent une valeur, ou score, représentant une distance à des objets de référence, que les classifications soient de type SVM ou non, ou plus généralement de type vectoriel ou non, comme par exemple les classifications baysiennes, les classifications linéaires, les classifications à base de réseau de neurones, les classification à base de distance tolérante, etc..

Dans une certaine mesure, il peut être argumenté qu'il n'existe pas encore d'outil fiable d'identification de micro-organismes à l'aide d'une spectrométrie de masse et d'outil de classification calculant des valeurs de distances.

## EXPOSE DE L'INVENTION

Le but de la présente invention est de résoudre le problème susmentionné en proposant un algorithme d'identification de micro-organismes à partir de mesures de spectres de masse et d'outil de classification, qui permettent d'identifier de manière plus fiable un micro-organisme.

A cet effet, l'invention a pour objet un procédé d'indentification par spectrométrie de masse d'un microorganisme parmi un groupe prédéterminé de microorganismes de référence, chaque microorganisme de référence étant représenté par un ensemble de données de référence, le procédé comportant :

■ la détermination d'un ensemble de données représentatives du microorganisme à identifier en fonction d'une mesure de spectrométrie de masse dudit microorganisme ; et
■ pour chaque microorganisme de référence, le calcul d'une distance entre ledit ensemble déterminé de données et l'ensemble de données de référence du microorganisme de référence.

Selon l'invention, le procédé comporte le calcul d'une probabilité que le microorganisme à identifier soit le microorganisme de référence, selon la relation :

$$f(m) = \frac{pN(m|\mu,\sigma)}{pN(m|\mu,\sigma) + (1-p)N(m|\overline{\mu},\overline{\sigma})}$$

où :

■ $m$ est la distance calculée pour le microorganisme de référence ;
■ $f(m)$ est la probabilité calculée pour ladite distance $m$ ;
■ $N(m|\mu,\sigma)$ est la valeur, pour la distance $m$, d'une variable aléatoire modélisant la distance entre un ensemble de données associé à un microorganisme à identifier et l'ensemble de données de référence du microorganisme de référence, lorsque le microorganisme à identifier est le microorganisme de référence ;
■ $N(m|\overline{\mu},\overline{\sigma})$ est la valeur, pour la distance $m$, d'une variable aléatoire modélisant la distance entre un ensemble de données associé à un microorganisme à identifier et l'ensemble de données de référence du microorganisme de référence, lorsque le microorganisme à identifier n'est pas le microorganisme de référence ; et
■ $p$ est un scalaire prédéterminé compris entre 0 et 1.

Par « ensemble de données de référence », on entend les données qui caractérise un microorganisme de référence au sens d'un outil de classification. Par exemple, pour une classification de type SVM-UTC, les données de référence associé à un microorganisme de référence correspondent à un hyperplan d'un espace vectoriel qui partitionne cet espace en deux sous-espace.

Par « ensemble de données représentatives », on entend les données utilisées pour caractériser un microorganisme à identifier au sens de l'outil de classification choisi. Par exemple, par la classification de type SVM-UTC, ces données sont une liste de pics détectés qui forme un vecteur de l'espace vectoriel.

En d'autres termes, l'invention s'applique à tout type de classification. Comme cela est connu en soi, une classification produit des distances, qui sont des quantités objectives mesurant des écarts à des éléments de référence. Selon l'invention ces distances sont transformées en des probabilités normalisées entre 0 et 1 selon une loi de type « sigmoïde ». Il en résulte que ces probabilités sont elles mêmes des quantités objectives comparables entre elles, et donc de réelles mesures de la « similarité » d'un microorganisme inconnu avec un microorganisme préalablement identifié.

Selon un mode de réalisation, les variables aléatoires $N(m|\mu,\sigma)$ et $N(m|\overline{\mu},\overline{\sigma})$ sont des variables aléatoires gaussiennes, de moyennes respectivement égales à $\mu$ et $\overline{\mu}$, et d'écarts types respectivement égaux à $\sigma$ et $\overline{\sigma}$. Plus particulièrement, la probabilité est calculée selon la relation :

$$f(m) = \frac{1}{1 + \frac{1-p}{p}\exp\left(\ln\left(\frac{\sigma}{\overline{\sigma}}\right) - \frac{1}{2(\sigma\overline{\sigma})^2}\left[(\sigma-\overline{\sigma})m - (\overline{\mu}\sigma - \mu\overline{\sigma})\right]\left[(\sigma+\overline{\sigma})m - (\overline{\mu}\sigma + \mu\overline{\sigma})\right]\right)}$$

De manière avantageuse, si la relation logique "$\mu > \overline{\mu}$" ⊕ "$\sigma > \overline{\sigma}$" est vérifiée, où ⊕ représente la fonction « ou exclusif », la probabilité est fixée à 1 pour toute distance $m$ supérieure à $\dfrac{\overline{\mu}\sigma^2 - \mu\overline{\sigma}^2}{\sigma^2 - \overline{\sigma}^2}$.

De manière avantageuse, si la relation logique "$\mu > \overline{\mu}$" ⊕ "$\sigma > \overline{\sigma}$" n'est pas vérifiée, où ⊕ représente la fonction « ou exclusif », la probabilité est fixée à 0 pour toute distance $m$ inférieure à $\dfrac{\overline{\mu}\sigma^2 - \mu\overline{\sigma}^2}{\sigma^2 - \overline{\sigma}^2}$.

Selon un mode de réalisation, le scalaire $p$ est identique pour tous les microorganismes de référence. Plus particulièrement, $p$ est égal à $\dfrac{1}{N}$, où $N$ est la taille de l'ensemble de microorganismes de référence. En variante, le scalaire p est égal à 0,5.

Selon un mode de réalisation de l'invention, la détermination du spectre de masse et le calcul de la distance entre le spectre acquis et chaque microorganisme de référence mettent en oeuvre un algorithme de classification vectorielle.

Plus particulièrement,

■ la détermination du spectre de masse du microorganisme de masse comporte :

○ l'acquisition d'au moins un spectre de masse dudit microorganisme ;
○ la détection de pics dans le au moins un spectre de masse acquis et la conversion des pics détectés en un vecteur d'un espace vectoriel prédéterminé,

■ et le calcul de distance entre ledit microorganisme et chaque microorganisme de référence comporte le calcul d'une distance algébrique entre le vecteur déterminé et une frontière qui partitionne l'espace vectoriel entre un premier sous-espace caractéristique du microorganisme de référence et un second sous-espace caractéristique des autres microorganismes de référence.

Notamment, la frontière d'un microorganisme de référence est calculée au moyen d'un algorithme de type *« support vector machine »* et d'un ensemble de vecteurs correspondants aux microorganismes de référence.

En variante, la détermination du spectre de masse et le calcul de la distance entre le spectre acquis et chaque microorganisme de référence mettent en oeuvre un algorithme de distance tolérante

Selon un mode de réalisation, le vecteur est calculé en répertoriant au plus un pic dans chaque intervalle d'une subdivision prédéterminée de la gamme des rapports masse-sur-charge du spectre de masse.

## BREVE DESCRIPTION DES FIGURES

L'invention sera mieux comprise à la lecture de la description qui suit, donnée uniquement à titre d'exemple, et réalisée en relation avec les dessins annexés, dans lesquels des références identiques désignent des éléments identiques ou analogues, et dans lesquels :

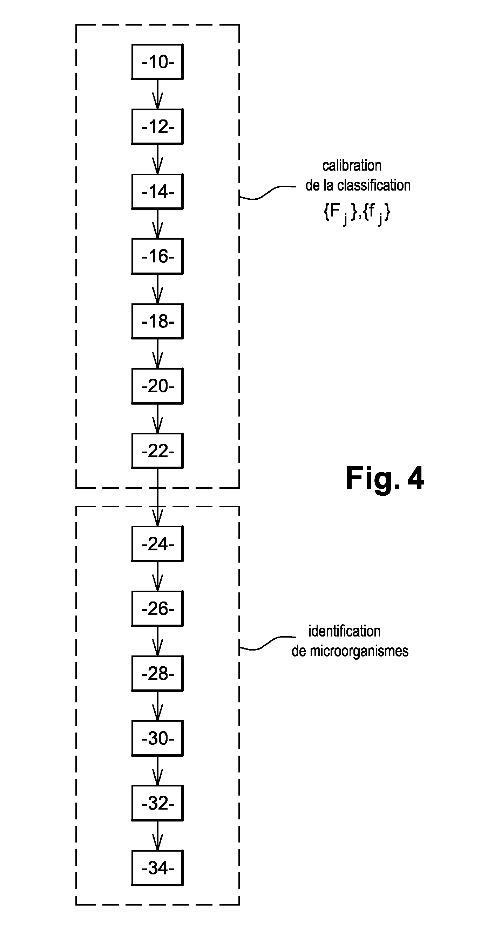

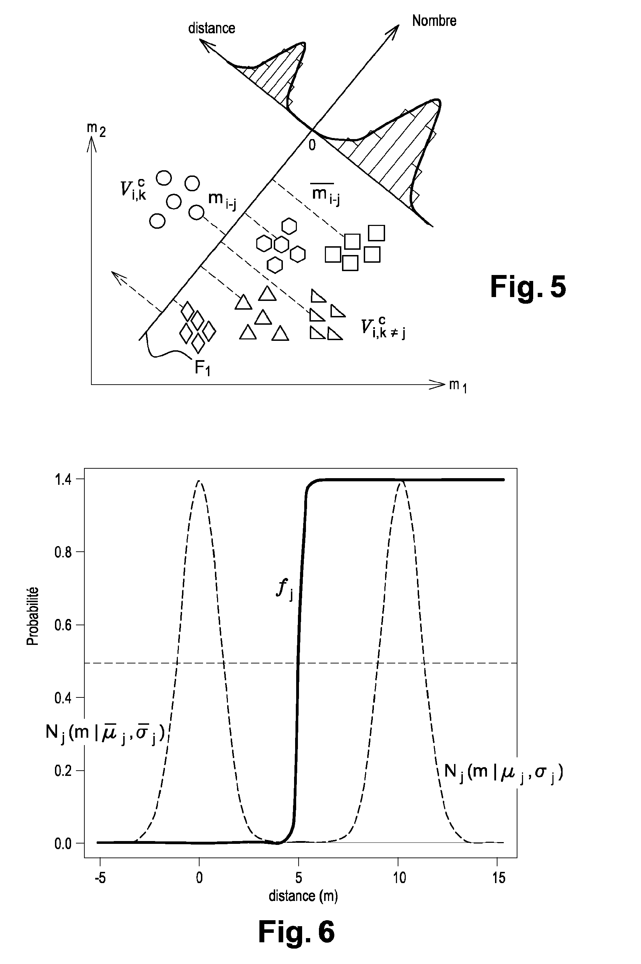

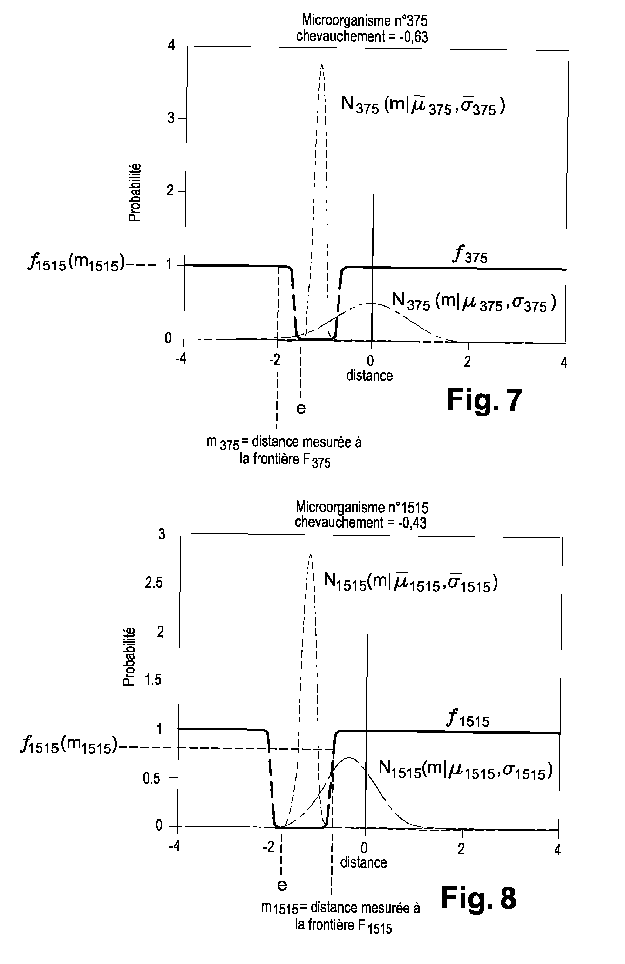

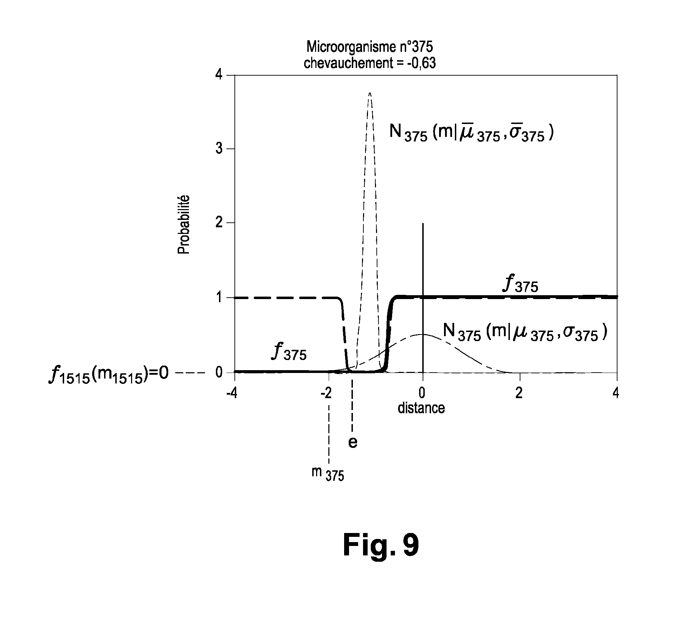

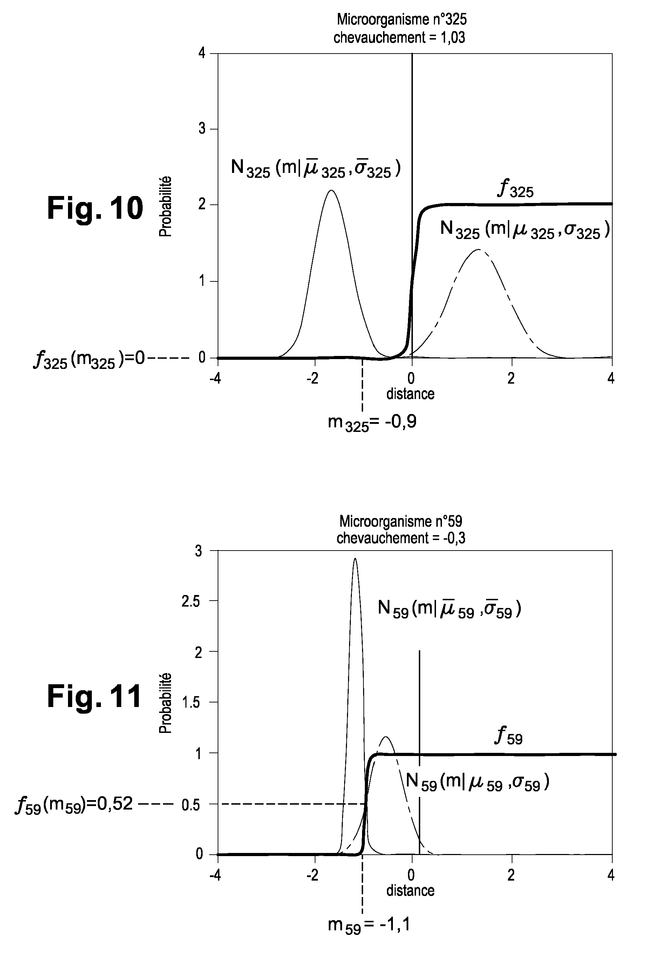

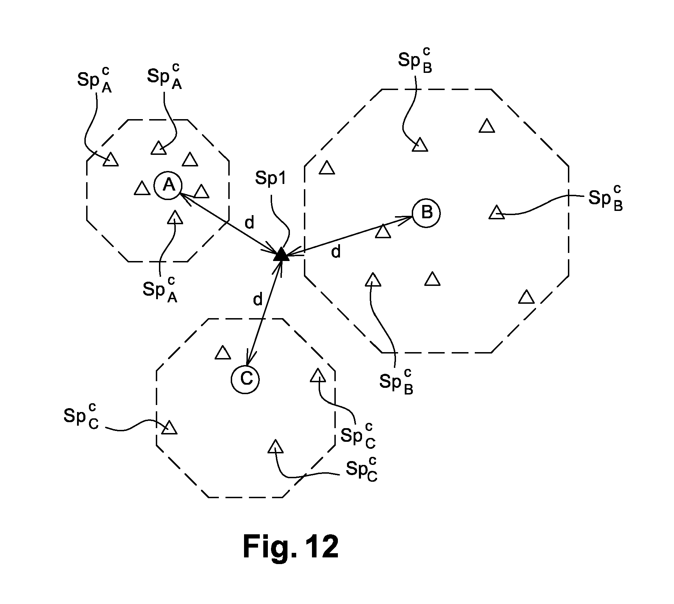

■ les figures 1A, 1B et 1C sont des exemples de spectre de masse à deux pics caractéristiques respectivement de 3 microorganismes ;
■ la figure 2 est un schéma illustrant l'obtention des trois frontières pour les organismes des figures 1A, 1B, 1C au moyen d'un algorithme de type SVM « un contre tous » ;
■ les figures 3A à 3D sont des schémas illustrant quatre distances d'un individu testé à une frontière de type SVM ;
■ la figure 4 est un organigramme d'un procédé selon l'invention ;
■ la figure 5 est un schéma illustrant la détermination de deux variables aléatoires à partir de distances de microorganismes à une frontière de type SVM ;
■ la figure 6 est un tracé illustrant deux distributions gaussiennes et une fonction de probabilité obtenues à partir de celles-ci ;
■ les figures 7 et 8 sont des tracés illustrant l'absence de caractère monotone pouvant survenir dans des fonctions de probabilité selon l'invention ;
■ la figure 9 est un tracé illustrant le résultat du forçage d'une fonction de probabilité selon l'invention à être monotone ;
■ les figures 10 et 11 sont des figures illustrant un cas d'identification difficile à l'aide de l'outil de classification et résolu par l'invention ; et
■ la figure 12 est un schéma illustrant des distances tolérantes d'un spectre à identifier avec des super-spectres selon l'état de la technique.

## DESCRIPTION DETAILLEE DE L'INVENTION

Il va à présent être décrit en relation avec l'organigramme de la figure 4, un procédé selon l'invention à base de classification SVM-UTC.

Le procédé débute par une étape **10** d'acquisition d'un ensemble de spectres de masse d'apprentissage et d'un ensemble de spectres de masse de calibration d'un nouveau microorganisme identifié à intégrer dans une base de connaissance, par exemple au moyen d'une spectrométrie de masse de type MALDI-TOF (acronyme de « *Matrix-assisted laser desorption/ionization time of flight* »). La spectrométrie de masse MALDI-TOF est bien connue en soi et ne sera donc pas décrite plus en détail par la suite. On pourra, par exemple, se référer au document de Jackson O. Lay, « Maldi-tof spectrometry of bacteria », Mass Spectrometry Reviews, 2001, 20, 172-194. Les spectres acquis sont ensuite prétraités, afin notamment de débruiter ceux-ci et ôter la leur ligne de base, d'une manière connue en soi.

Une identification des pics présents dans les spectres acquis est alors réalisée en **12,** par exemple au moyen d'un algorithme de détection de pics basé sur la détection de maxima locaux. Une liste des pics pour chaque spectre acquis, comportant la localisation et l'intensité des pics du spectre, est ainsi produite.

De manière avantageuse, les pics sont identifiés dans la gamme de Thomson $[m_{min} ; m_{max}]$ prédéterminée, de préférence la gamme $[m_{min} ; m_{max}]=[3000;17000]$ Thomson. En effet, il a été observé que les informations suffisantes à l'identification des micro-organismes sont regroupées dans cette gamme de rapport masse sur charge, et qu'il n'est donc pas besoin de tenir compte d'une gamme plus large.

Le procédé se poursuit, en **14,** par une étape de quantification, ou « binning ». Pour ce faire la gamme $[m_{min}; m_{max}]$ est subdivisée en intervalles de largueur, par exemple constante, et pour chaque intervalle comprenant plusieurs pics, un seul pic est conservé, avantageusement le pic présentant la plus forte intensité. Un vecteur est ainsi produit pour chaque spectre mesuré. Chaque composante du vecteur correspond à un intervalle de la quantification et a pour valeur l'intensité du pic conservé pour cet intervalle, la valeur « 0 » signifiant qu'aucun pic n'a été détecté dans cet intervalle.

En variante, les vecteurs sont « binarisés » en posant la valeur d'une composante du vecteur à « 1 » lorsqu'un pic est présent dans l'intervalle correspondant, et à « 0 » lorsqu'aucun pic n'est présent dans cet intervalle. Ceci a pour effet de rendre plus robuste la calibration des algorithmes de classification réalisée ultérieurement. Les inventeurs ont en effet noté que l'information pertinente notamment pour l'identification d'une bactérie est contenue essentiellement dans l'absence et/ou la présence de pics, et que l'information d'intensité est moins pertinente. En outre, on observe que l'intensité est une grandeur très variable d'un spectre à l'autre et/ou d'un spectromètre à un autre. Du fait de cette variabilité, il est difficile de prendre en compte les valeurs brutes d'intensité dans les outils de classification.

Parallèlement, les vecteurs de pics de spectres d'apprentissage, ci-après « vecteurs d'apprentissage », et les vecteurs de pics des spectres de calibration, ci-après « vecteurs de calibration », sont mémorisés dans la base de connaissance. Pour chacun des microorganismes enregistrés dans la base de connaissance, il existe donc un premier ensemble $\left\{V_{i,j}^{a}\right\}$ de vecteurs d'apprentissage $V_{i,j}^{a}$, et un second ensemble $\left\{V_{i,j}^{c}\right\}$ de vecteurs de calibration $V_{i,j}^{c}$.

Dans une étape **16** suivante, une frontière $F_j$ est calculée pour chaque microorganisme $j$ de la base de connaissance entre l'ensemble des vecteurs d'apprentissage $\left\{V_{i,j}^{a}\right\}$ du microorganisme et l'ensemble $\left\{V_{i,k\neq j}^{a}\right\}$ des vecteurs d'apprentissage $V_{i,k\neq j}^{a}$ des autres microorganismes. Cette frontière $F_j$ est calculée au moyen d'un algorithme de classification SVM-UTC. Cet algorithme est classique et pour plus de détails on pourra par exemple se reporter au document de R.-E. Fan, K.-W. Chang, C.-J. Hsieh, X.-R. Wang, and C.-J. Lin. « LIBLINEAR: A Library for Large Linear Classification », Journal of Machine Learning Research 9(2008), 1871-1874. Un logiciel codant cet algorithme est par exemple accessible à l'adresse http://www.csie.ntu.edu.tw/~cjlin/liblinear.

Le procédé consiste ensuite à déterminer une fonction $f_j$ qui transforme la distance algébrique à une frontière $F_j$ en un degré de similarité avec le microorganisme $j$ associé à cette frontière $F_j$. Le principe sous-jacent à cette transformation est de considérer la distance d'un microorganisme $j$ à sa frontière $F_j$ associée comme étant une première variable aléatoire $N_j(m|\mu_j,\sigma_j)$, et la distance des autres microorganismes à cette frontière comme étant une seconde variable aléatoire $N_j(m|\overline{\mu}_j,\overline{\sigma}_j)$, puis de déterminer une fonction $f_j$ monotone, variant de 0 à 1, qui sépare les deux variables aléatoires $N_j(m|\mu_j,\sigma_j)$ et $N_j(m|\overline{\mu}_j,\overline{\sigma}_j)$.

Le procédé se poursuit donc par l'identification, en **18,** de ces deux variables aléatoires $N_j(m|\mu_j,\sigma_j)$ et $N_j(m|\overline{\mu}_j, \overline{\sigma}_j)$ pour chaque microorganisme $j$ de la base de connaissance, en fonction des vecteurs de calibration $\left\{V_{i,j}^{c}\right\}$ et $\left\{V_{i,k\neq j}^{c}\right\}$.

En utilisant des vecteurs de calibration différents des vecteurs d'apprentissage utilisés pour calculer les frontières $F_j$, on évite un phénomène de sur-apprentissage qui peut être nuisible à la robustesse et la précision de l'identification. Bien entendu, il est également possible d'utiliser les vecteurs d'apprentissage pour calculer les fonctions $f_j$.

En reprenant l'exemple des figures 1 et 2 dans lequel des microorganismes sont identifiables par la localisation de deux pics du spectre de masse, le calcul de ces variables aléatoire est illustré à la figure 5 dans laquelle sont représentés des vecteurs de calibration $V_{i,j}^{c}$ d'un microorganisme $j$, séparés par une frontière $F_j$ des vecteurs de calibration $V_{i,k\neq j}^{c}$ des autres microorganismes de la base de connaissance.

Tout d'abord, il est calculé la distance $m_{i,j}$ entre chaque vecteur de calibration $V_{i,j}^{c}$ du microorganisme $j$ et la

7

frontière $F_j$, ainsi que la distance $\overline{m_{i,j}}$ entre chaque vecteur de calibration $V_{i,k\neq j}^c$ des autres microorganismes de la base de connaissance à ladite frontière $F_j$.

Ensuite, la variable aléatoire $N_j(m|\mu_j,\sigma_j)$ modélisant la distance entre un microorganisme j et sa frontière $F_j$ est identifiée à partir de l'ensemble des distances $m_{i,j}$ et la variable aléatoire $N_j(m|\overline{\mu}_j,\overline{\sigma}_j)$ modélisant la distance entre les autres microorganismes et cette frontière $F_j$ est identifiée à partir de l'ensemble des distances $\overline{m}_{i,j}$

De manière avantageuse, le modèle de variable aléatoire choisi est une distribution gaussienne. Les variables aléatoires $N_j(m|\mu_j,\sigma_j)$ et $N_j(m|\overline{\mu}_j,\overline{\sigma}_j)$ s'écrivent donc selon les relations :

$$N_j\left(m\middle|\mu_j,\sigma_j\right)=\frac{1}{\sigma_j\sqrt{2\pi}}\cdot\exp\left(-\frac{1}{2}\left(\frac{m-\mu_j}{\sigma_j}\right)^2\right) \tag{1}$$

$$N_j\left(m\middle|\overline{\mu}_j,\overline{\sigma}_j\right)=\frac{1}{\overline{\sigma}_j\sqrt{2\pi}}\cdot\exp\left(-\frac{1}{2}\left(\frac{m-\overline{\mu}_j}{\overline{\sigma}_j}\right)^2\right) \tag{2}$$

$$\mu_j=\frac{1}{M_j^c}\sum_{i=1}^{M_j^c}m_{i,j} \tag{3}$$

$$\sigma_j=\sqrt{\frac{1}{M_j^c-1}\sum_{i=1}^{M_j^c}\left(m_{i,j}-\mu_j\right)^2} \tag{4}$$

$$\overline{\mu}_j=\frac{1}{M_j^{cneg}}\sum_{i=1}^{M_j^{cneg}}\overline{m}_{i,j} \tag{5}$$

$$\overline{\sigma}_j=\sqrt{\frac{1}{M_j^{cneg}-1}\sum_{i=1}^{M_j^{cneg}}\left(\overline{m}_i,j-\overline{\mu}_j\right)^2} \tag{6}$$

où $m$ est la distance à la frontière $F_j$, $M_j^c$ est le nombre de vecteurs de calibration $V_{i,j}^c$ du microorganisme j, et $M_j^{cneg}$ est le nombre total des vecteurs de calibration $V_{i,k\neq j}^c$ des autres microorganismes de la base de connaissance.

Le procédé se poursuit par le calcul, en 20, d'une fonction $f_j$ de probabilité pour chaque microorganisme j qui sépare les distributions $N_j(m|\mu_j,\sigma_j)$ et $N_j(m|\overline{\mu}_j,\overline{\sigma}_j)$, tel qu'illustrée à la figure 6.

Plus particulièrement, la fonction $f_j$ satisfait la relation :

$$f_j(m)=\frac{p_j\times N_j\left(m\middle|\mu_j,\sigma_j\right)}{p_j\times N_j\left(m\middle|\mu_j,\sigma_j\right)+\left(1-p_j\right)\times N_j\left(m\middle|\overline{\mu}_j,\overline{\sigma}_j\right)} \tag{7}$$

où $p_j$ est un paramètre de réglage prédéterminé relatif à une information a priori sur la prévalence du microorganisme $j$.

Le paramètre $p_j$ est par exemple égal à 0,5 si l'événement « la distance $m$ correspond au microorganisme $j$ » a le même poids que l'évènement « la distance $m$ correspond à un microorganisme différent du microorganisme $j$ ». Dans cette hypothèse, on considère que si $m$ se trouve à équidistance des 2 distributions, le spectre a une probabilité de $\dfrac{1}{2}$ d'être le micro-organisme de référence j, et par conséquent une probabilité de $\dfrac{1}{2(N-1)}$ d'être n'importe lequel des N-1 autres micro-organismes de référence, où $N$ est le nombre de microorganismes enregistrés dans la base de connaissances.

En variante, le paramètre $p_j$ est égal à $\dfrac{1}{N}$, où $N$ est le nombre de microorganismes enregistrés dans la base de connaissances. Dans cette hypothèse on considère que si $m$ se trouve à équidistance des 2 distributions, le spectre à la même probabilité d'être n'importe lequel des micro-organismes de références.

Comme cela est illustrée à la figure 6, la fonction de probabilité $f_j$ est une fonction monotone au moins sur un intervalle de distances comprenant l'essentiel des distributions $N_j(m|\mu_j,\sigma_j)$ et $N_j(m|\overline{\mu}_j,\overline{\sigma}_j)$, et qui croit de 0 à 1 en prenant la forme d'une sigmoïde. Notamment, pour les valeurs importantes de $N_j(m|\overline{\mu}_j,\sigma_j)$, cette fonction est égale à 0 alors que pour les valeurs importantes de $N_j(m|\mu_j,\sigma_j)$ cette fonction est égale à 1.

La fonction $f_j$ représente en effet un degré de vraisemblance, qu'un microorganisme ayant une *distance m* à la frontière $F_j$ soit le microorganisme $j$. Plus particulièrement, on montre que la fonction $f_j$ correspond à la probabilité $p(s|m)$ qu'un microorganisme testé soit le microorganisme $j$ lorsque l'on observe la distance $m$, c'est-à-dire que la fonction $f_j$ selon la relation 7 correspond à la probabilité $p(s|m)$ selon les relations :

$$p(s|m) = \frac{p(s)p(m|s)}{p(m)} \tag{8}$$

$$p(m) = p(s)p(m|s) + p(\overline{s})p(m|\overline{s}) \tag{9}$$

où $p(s)$ est une distribution a priori sur la probabilité d'obtenir le microorganisme $j$, c'est-à-dire sa prévalence, $p(m|s)$ est la vraisemblance de *distance m* pour le microorganisme $j$, et $p(m|\overline{s})$ est la vraisemblance de la *distance m* pour les autres microorganismes. Selon l'invention, on a donc $p(s) = p_j$, $p(m|s) = N_j(m|\mu_j,\sigma_j)$ et $p(m|\overline{s}) = N_j(m,|\overline{\mu}_j,\overline{\sigma}_j)$.

Si le cas des distributions exposées à la figure 6 est un cas standard, il peut parfois arriver que la fonction $f_j$ ne soit pas monotone sur une partie extrême d'une distribution, comme cela est représenté aux figures 7 et 8.

En effet, le modèle de distribution $N_j(m|\mu_j,\sigma_j)$ et $N_j(m|\overline{\mu}_j,\overline{\sigma}_j)$ choisi peut être imprécis, ou le nombre d'échantillons utilisés pour générer les vecteurs de calibration $\left\{V_{i,j}^c\right\}$ et $\left\{V_{i,k\neq j}^c\right\}$ être limité, et donc induire une incertitude sur les paramètres des distributions, ou encore des valeurs aberrantes de distance peuvent être produites, par exemple en raison d'incertitudes de mesure.

Notamment, la fonction de transformation en probabilités $f_j$ calculée à partir de lois normales tels que définies aux relations (1) à (6) peut ne pas être monotone. En particulier, les deux distributions normales $N_j(m|\mu_j,\sigma_j)$ et $N_j(m|\overline{\mu}_j,\overline{\sigma}_j)$ ont des écart-types $\sigma_j$ et $\overline{\sigma}_j$ le plus souvent très différents, la distribution $N_j(m|\mu\overline{\mu}_j,\overline{\sigma}_j)$ associée aux non-représentants du microorganisme $j$ étant généralement bien plus étroite que la distribution $N_j(m|\mu_j,\sigma_j)$ des représentants du microorganisme $j$. Ceci se traduit souvent par un caractère non monotone de la fonction $f_j$ sur l'ensemble des distances, notamment par une remontée de la fonction $f_j$ pour les distances à « gauche » de la distribution $N_j(m|\overline{\mu}_j,\overline{\sigma}_j)$, comme illustré sur les figures 7 et 8.

Par exemple sur la figure 7, pour une distance $m_{375}$ entre un microorganisme à identifier et la frontière $F_{375}$ associée au microorganisme n°375 de la base de connaissance égale à -2, sans mesure particulière, la fonction $f_{375}$ du microorganisme n°375 est égale à 1. Ceci signifie que le microorganisme à identifier est le microorganisme n°375 avec une probabilité proche de 100%, bien qu'il apparaisse au contraire que la probabilité réelle que le microorganisme mesuré soit le microorganisme n°375 est très faible. En outre, il existe d'autres fonctions $f_j$ pour lesquelles la valeur sera positive pour la *distance m* du microorganisme à identifier à la frontière $F_j$ associée au microorganisme $j$, et donc une probabilité associée bien supérieure à 0. Par exemple sur la figure 8, la probabilité $f_{375}(m_{375})$ du microorganisme à identifier, associée à la distance $m_{375}$ de ce dernier à la frontière $F_{1515}$ d'un microorganisme n°1515 de la base de connaissance, est égale à 0,9, soit une probabilité inférieure à celle associée au microorganisme n°375, alors qu'il apparaît que le microorganisme à identifier est effectivement le microorganisme n°1515. Il convient donc que la confiance en cette identification au microorganisme n°1515 soit supérieure à celle associée au microorganisme n°375.

Bien que dans la réalité, il est peu probable, d'obtenir des distances mesurées dans des parties extrémales des fonctions $f_j$ où il pourrait exister un tel comportement, la fonction $f_j$ est avantageusement déterminée de manière à garantir son caractère monotone.

On notera à cet égard que les distributions gaussiennes sont avantageusement choisies en raison de la simplicité de leur calcul, et que le comportement non monotone qui peut dans certains cas résulter de ce choix n'est pas critique. En effet, l'utilisation de gaussienne présente l'avantage de produire des fonctions $f_j$ dont le comportement est analytiquement prédictible. Il est donc possible de modifier les fonctions $f_j$ selon la relation (7) de manière à garantir en sortie un comportement monotone sur l'ensemble des distances.

Plus particulièrement, on montre que pour une fonction $f_j$ à base de distributions gaussiennes, il ne peut exister qu'un seul minimum local de valeur selon la relation :

$$e = \frac{\overline{\mu}_j \sigma_j^2 - \mu_j \overline{\sigma}_j^2}{\sigma_j^2 - \overline{\sigma}_j^2} \qquad (10)$$

De manière avantageuse, le procédé se poursuit donc par une étape 22 dans laquelle les fonctions $f_j$ sont rendues monotones. Plus particulièrement, il est posé :

$$f_j(m) = 0 \text{ pour tout } m \leq e, \text{ si } \mu > \overline{\mu} \text{ , ou de manière équivalente } \sigma > \overline{\sigma} \qquad (11)$$

$$f_j(m) = 1 \text{ pour tout } m \geq e, \text{ si la relation logique } "\mu > \overline{\mu}" \oplus "\sigma > \overline{\sigma}" \text{ est vraie} \qquad (12)$$

où $\oplus$ est le symbole de la fonction logique « ou exclusif ».

Une application de l'étape 22 est par exemple illustrée à la figure 9 dans laquelle la fonction $f_{375}$ illustrée à la figure 7 est forcée à la valeur nulle pour toute distance inférieure à la valeur e, de sorte que la fonction $f_{375}$ est à présent une fonction monotone de type sigmoïde sur l'ensemble des distances.

A l'issu de l'étape 22, l'outil de classification selon l'invention est donc calibré. Les frontières $F_j$ de l'outil de classification SVM-UCT ainsi que les fonctions de probabilité $f_j$ sont mémorisées dans la base de connaissances.

La base de connaissance est incorporée dans un système d'identification de microorganismes par spectrométrie de masse comprenant un spectromètre de masse, par exemple un spectromètre de type MALDI-TOF, ainsi qu'une unité de traitement d'informations, connectée au spectromètre et apte à recevoir et traiter les spectres de masse acquis pour identifier des microorganismes inconnus.

Plus particulièrement, pour l'identification d'un microorganisme inconnu, le procédé comporte une étape 24 d'acquisition d'un ou plusieurs spectres de masse de celui-ci, une étape 26 de prétraitement des spectres acquis ainsi qu'une étape 28 de détection des pics des spectres et de détermination d'un vecteur de pics $V_m$, tel que par exemple décrites précédemment en relation avec les étapes 10 à 14.

Dans une étape 30 suivante, la distance $m_j$ du vecteur $V_m$ à chacune des frontières $F_j$ est calculée, puis chacune des distances $m_j$ est transformée, dans l'étape 32, en la probabilité correspondante $f_j(m_j)$.

Dans une première variante, les probabilités $f_j(m_j)$ sont calculées numériquement selon les formules (1), (2) et (7), à savoir selon la relation du type :

$$f_j(m) = \frac{p_j \times \dfrac{1}{\sigma_j\sqrt{2\pi}} \cdot \exp\left(-\dfrac{1}{2}\left(\dfrac{m-\mu_j}{\sigma_j}\right)^2\right)}{p_j \times \dfrac{1}{\sigma_j\sqrt{2\pi}} \cdot \exp\left(-\dfrac{1}{2}\left(\dfrac{m-\mu_j}{\sigma_j}\right)^2\right) + (1-p_j) \times \dfrac{1}{\overline{\sigma}_j\sqrt{2\pi}} \cdot \exp\left(-\dfrac{1}{2}\left(\dfrac{m-\overline{\mu}_j}{\overline{\sigma}_j}\right)^2\right)} \qquad (13)$$

Dans cette variante, le numérateur et le dénominateur sont calculées indépendamment l'un de l'autre en mettant en oeuvre des fonctions exponentielles numériques. Toutefois, lorsqu'un microorganisme présente une distance $m_j$ loin des moyennes $\mu_j$ et $\overline{\mu}_j$ des deux distributions gaussiennes, il existe un risque que les exponentielles numériques résultantes soient approximées à 0 et qu'il en résulte une indétermination du type 0/0 pour la valeur $f_j(m_j)$.

Selon une seconde variante, les probabilités $f_j(m_j)$ sont calculées selon la relation (14) suivante :

$$f_j(m) = \frac{1}{1 + \dfrac{1-p_j}{p_j}\exp\left(\ln\left(\dfrac{\sigma_j}{\overline{\sigma}_j}\right) - \dfrac{1}{2(\sigma_j\overline{\sigma}_j)^2}\left[(\sigma_j-\overline{\sigma}_j)m_j - (\overline{\mu}_j\sigma_j - \mu_j\overline{\sigma}_j)\right]\left[(\sigma_j+\overline{\sigma}_j)m_j - (\overline{\mu}_j\sigma_j + \mu_j\overline{\sigma}_j)\right]\right)}$$

La relation (13) est du point de vue mathématique strictement équivalente à la relation (14). Toutefois, la relation (14) est plus robuste aux approximations numériques, et supporte même des distances de valeur infinie sans produire d'indétermination.

Dans le cas où les fonctions $f_j$ sont forcées monotones, les relations (11) et (12) sont également appliquées.

Les probabilités $f_j(m_j)$ sont alors classées par ordre décroissant. Si aucune ne dépasse un certain seuil, on considère par exemple qu'il n'y a pas d'identification. Si au contraire une ou plusieurs dépassent un certain seuil, elles sont par exemple affichées, en **34,** sur un écran du système d'identification avec une liste de microorganismes associés Par exemple une liste de 3 microorganismes, est affichée avec leur valeur de $f_j(m_j)$ correspondante qui constitue directement le degré de confiance que l'on peut accorder à l'identification des microorganismes.

Les figures 10 et 11 illustrent un cas d'identification particulièrement difficile d'un microorganisme par l'outil de classification et résolu par l'invention. La figure 10 est un tracé illustrant des distributions gaussiennes $N_{325}(m|\mu_{325}, \sigma_{325})$ et $N_{325}(m|\overline{\mu}_{325}, \overline{\sigma}_{325})$, ainsi que leur fonctions $f_{325}$ associées, correspondant à un organisme n°325 de la base de connaissance, et la figure 11 est un tracé illustrant des distributions gaussiennes $N_{59}(m|\mu_{59}, \sigma_{59})$ et $N_{59}(m|\overline{\mu}_{59}, \overline{\sigma}_{59})$, ainsi que leur fonctions $f_{59}$, correspondant à un organisme n°59 de la base de connaissance. Une distance $m_{59}$ d'un microorganisme à identifier à la frontière $F_{59}$ du microorganisme n°59 est égale à - 1,1, alors que la distance $m_{325}$ du microorganisme à identifier à la frontière $F_{325}$ du microorganisme n°325 est égale à - 0,9.

Conformément à l'état de la technique, qui se fonde sur la comparaison directe des distances, sans analyse supplémentaire, il en est donc conclu que le microorganisme à identifier est le microorganisme n°325. Or, en regardant les distributions, on voit que le microorganisme à identifier n'a pratiquement aucune chance d'être le microorganisme n°325, alors qu'il a de bonnes chances d'être le microorganisme n°59. Seule une analyse supplémentaire, basée sur le savoir faire de la personne en charge de l'identification, est capable de conclure à partir des distances mesurées que c'est en fait le microorganisme n°59 qui a été identifié.

La transformation des distances en fonction de probabilité selon l'invention permet de résoudre ce type de cas complexe. En effet, la fonction $f_{325}(m_{325})$ est nulle, signifiant que la probabilité que le microorganisme à identifier soit le microorganisme n°325 est nulle, alors que la fonction $f_{59}(m_{59})$ est positive, ici égale à 0,52, signifiant que le microorganisme à identifier est le microorganisme n°59 avec une confiance de 52%.

Il a été décrit un mode de réalisation particulier de l'invention dans lequel des distributions gaussiennes sont utilisées pour modéliser les variables aléatoires $N_j(m|\mu_j, \sigma_j)$ et $N_j(m|\overline{\mu}_j, \overline{\sigma}_j)$.

D'autres types de variables aléatoires sont bien entendues possibles, l'essentiel étant d'obtenir une modélisation pertinente des distributions. Il est par exemple possible de choisir des mélanges de gaussiennes, pour tenir compte de la présence éventuelle de plusieurs modalités dans les distributions. Les distributions gaussiennes sont avantageuses dans la mesure où leur identification est très simple et qu'il a été constaté que cette modélisation, même imparfaite, permettait l'obtention de fonctions de probabilité $f_j$ robustes.

De même, il a été décrit un mode de réalisation dans lequel l'outil de classification utilisé est un algorithme de type SVM « un contre tous ».

Bien entendu l'invention n'est pas limitée à ce type d'algorithme et s'applique à tout type d'algorithme de classification, notamment aux algorithmes de classification multi-classes réalisés par combinaison de classifications binaires de type « un contre tous » ou « tous contre tous » par exemple, dès lors que l'algorithme de classification produit une valeur, ou score, représentant une distance à des objets de référence, qui n'est pas directement un indice de confiance.

Notamment, l'invention s'applique à des algorithmes de classification appliquant un calcul de similitude par rapport à des éléments référence, comme par exemple des spectres moyens, ou des « super-spectres » tels que décrits dans le document EP 1 253 622.

Dans l'algorithme décrit dans le document EP 1 253 622 B1, des « spectres de référence synthétique » (REFs), appelé également « super-spectres » sont construits et correspondent chacun à une liste de pics réputés les plus typique d'une espèce donnée. Pour identifier un microorganisme à l'aide d'un spectre de masse, il est calculé une « similitude » de ce spectre mesuré à tous les super-spectres mémorisés dans une base de connaissance. Cette similitude peut par exemple être une distance intégrant une tolérance sur les masses, comme par exemple une distance de Jaccard ou de Hamming.

Notamment, si *SSp1* est un super-spectre composé d'une liste de masses $M_i$ et de poids associés $W_i$, et $Sp_1$ est une liste de masses $M'_j$ associées à des pics détecté dans le spectre de masse du microorganisme à identifier, alors la similitude, ou distance tolérante, entre *SSp1* et *Sp1* est calculée, comme cela est connu en soi, selon la relation :

$$s(SSp1/Sp1) = \frac{\sum W_i, \exists M'_j, \frac{\left| M'_j - M_i \right|}{M_i} < d}{\sum W_i} \qquad (15)$$

où *d* est la tolérance relative acceptée entre deux masses.

Selon l'état de la technique, on retient comme identification l'organisme correspondant au super-spectre ayant la similitude la plus forte, cette valeur de similitude servant directement de mesure de la confiance qu'on peut avoir dans le résultat. Or, selon le choix des pics qui ont servi à construire le super-spectre, les similitudes moyennes pour l'ensemble des spectres d'une espèce avec leur super-spectre (REFs) ne sont pas forcément les mêmes. Ainsi, dans l'exemple illustré à la figure 12, bien que le spectre *Sp1* soit à équidistance des 3 super-spectres *A, B, C*, on comprend que la probabilité qu'il soit effectivement de l'espèce A est inférieure à celle qu'il soit de l'espèce B, comme cela apparaît clairement à la vue de la répartition des spectres échantillons $\left\{ Sp_A^c \right\}$, $\left\{ Sp_B^c \right\}$, $\left\{ Sp_C^c \right\}$ ayant servis de base à la construction des super-spectres. L'invention permet de normaliser les similitudes obtenues et ainsi de lever cette ambigüité.

**Revendications**

1. Procédé d'indentification par spectrométrie de masse d'un microorganisme parmi un ensemble prédéterminé de microorganismes de référence, chaque microorganisme de référence étant représenté par un ensemble de données de référence, le procédé comportant :

   ■ la détermination d'un ensemble de données représentatives du microorganisme à identifier en fonction d'une mesure de spectrométrie de masse dudit microorganisme ; et
   ■ pour chaque microorganisme de référence, le calcul d'une distance entre ledit ensemble déterminé de données et l'ensemble de données de référence du microorganisme de référence,
   *caractérisé* **en ce qu'**il comporte le calcul d'une probabilité que le microorganisme à identifier est le microorganisme de référence, selon la relation :

$$f(m) = \frac{pN(m|\mu,\sigma)}{pN(m|\mu,\sigma) + (1-p)N(m|\overline{\mu},\overline{\sigma})}$$

où :

■ $m$ est la distance calculée pour le microorganisme de référence ;

■ $f(m)$ est la probabilité calculée pour ladite distance $m$ ;

■ $N(m|\mu,\sigma)$ est la valeur, pour la distance $m$, d'une variable aléatoire modélisant la distance entre un ensemble de données associé à un microorganisme à identifier et l'ensemble de données de référence du microorganisme de référence, lorsque le microorganisme à identifier est le microorganisme de référence ;

■ $N(m|\overline{\mu},\sigma)$ est la valeur, pour la distance $m$, d'une variable aléatoire modélisant la distance entre un ensemble de données associé à un microorganisme à identifier et l'ensemble de données de référence du microorganisme de référence, lorsque le microorganisme à identifier n'est pas le microorganisme de référence ; et

■ $p$ est un scalaire prédéterminé compris entre 0 et 1.

2. Procédé d'identification selon la revendication 1, *caractérisé* **en ce que** les variables aléatoires $N(m|\mu,\sigma)$ et $N(m|\overline{\mu},\overline{\sigma})$ sont des variables aléatoires gaussiennes, de moyennes respectivement égales à $\mu$ et $\overline{\mu}$, et d'écarts types respectivement égaux à $\sigma$ et $\overline{\sigma}$.

3. Procédé d'identification selon la revendication 2, *caractérisé* **en ce que** la probabilité est calculée selon la relation :

$$f(m) = \frac{1}{1 + \frac{1-p}{p}\exp\left(\ln\left(\frac{\sigma}{\overline{\sigma}}\right) - \frac{1}{2(\sigma\overline{\sigma})^2}\left[(\sigma - \overline{\sigma})m - (\overline{\mu}\sigma - \mu\overline{\sigma})\right]\left[(\sigma + \overline{\sigma})m - (\overline{\mu}\sigma + \mu\overline{\sigma})\right]\right)}$$

4. Procédé d'identification selon la revendication 2 ou 3, *caractérisé* **en ce que**, si la relation logique "$\mu > \overline{\mu}$" $\oplus$ "$\sigma > \overline{\sigma}$" est vérifiée, où $\oplus$ représente la fonction « ou exclusif », la probabilité est fixée à 1 pour toute distance $m$ supérieure à $\dfrac{\overline{\mu}\sigma^2 - \mu\overline{\sigma}^2}{\sigma^2 - \overline{\sigma}^2}$ .

5. Procédé d'indentification selon la revendication 2 ou 3, *caractérisé* **en ce que** si la relation logique "$\mu > \overline{\mu}$" $\oplus$ "$\sigma > \overline{\sigma}$" n'est pas vérifiée, où $\oplus$ représente la fonction « ou exclusif », la probabilité est fixée à 0 pour toute distance $m$ inférieure à $\dfrac{\overline{\mu}\sigma^2 - \mu\overline{\sigma}^2}{\sigma^2 - \overline{\sigma}^2}$ .

6. Procédé d'indentification selon l'une quelconque des revendications précédentes, **caractérisé en ce que** le scalaire $p$ est identique pour tous les microorganismes de référence.

7. Procédé d'indentification selon la revendication 6, *caractérisé* **en ce que** le scalaire $p$ est égal à $\dfrac{1}{N}$ , où $N$ est la taille de l'ensemble de microorganismes de référence.

8. Procédé d'indentification selon l'une quelconque des revendications 6, *caractérisé* le scalaire $p$ est égal à 0,5.

9. Procédé d'indentification selon l'une quelconque des revendications précédentes, *caractérisé* **en ce que** la détermination du spectre de masse et le calcul de la distance entre le spectre acquis et chaque microorganisme de référence mettent en oeuvre un algorithme de classification vectorielle.

10. Procédé d'indentification selon la revendication 9, *caractérisé :*

■ en ce que la détermination du spectre de masse du microorganisme de masse comporte :

o l'acquisition d'au moins un spectre de masse dudit microorganisme ;
o la détection de pics dans le au moins un spectre de masse acquis et la conversion des pics détectés en un vecteur d'un espace vectoriel prédéterminé,

■ et en ce que le calcul de distance entre ledit microorganisme et chaque microorganisme de référence comporte le calcul d'une distance algébrique entre le vecteur déterminé et une frontière qui partitionne l'espace vectoriel entre un premier sous-espace caractéristique du microorganisme de référence et un second sous-espace caractéristique des autres microorganismes de référence.

**11.** Procédé d'indentification selon la revendication 9 ou 10, *caractérisé* **en ce que** la frontière d'un microorganisme de référence est calculée au moyen d'un algorithme de type « support vector machine » et d'un ensemble de vecteurs correspondants aux microorganismes de référence.

**12.** Procédé d'indentification selon l'une des revendications 9, 10 ou 11, *caractérisé* **en ce que** le vecteur est calculé en répertoriant au plus un pic dans chaque intervalle d'une subdivision prédéterminée de la gamme des rapports masse-sur-charge du spectre de masse.

**13.** Procédé d'indentification selon l'une quelconque des revendications précédentes, *caractérisé* **en ce que** la détermination du spectre de masse et le calcul de la distance entre le spectre acquis et chaque microorganisme de référence mettent en oeuvre un algorithme de distance tolérante.

**14.** Dispositif d'identification d'un microorganisme par spectrométrie de masse, comprenant :

■ un spectromètre de masse apte à produire des spectres de masse de microorganismes à identifier ;
■ une unité de calcul apte à identifier les microorganismes associés aux spectres de masse produits par le spectromètre en mettant en oeuvre un procédé conforme à l'une quelconque des revendications précédentes.

Fig. 1A

Fig. 1B

Fig. 1C

Fig. 2

Fig. 3A

Fig. 3B

Fig. 3C

Fig. 3D

calibration
de la classification

$\{F_j\},\{f_j\}$

**Fig. 4**

identification
de microorganismes

**Fig. 5**

**Fig. 6**

Microorganisme n°375
chevauchement = -0,63

$N_{375}(m|\bar{\mu}_{375},\bar{\sigma}_{375})$

$f_{1515}(m_{1515})$--- 1

$f_{375}$

$N_{375}(m|\mu_{375},\sigma_{375})$

distance

**Fig. 7**

$m_{375}$ = distance mesurée à
la frontière $F_{375}$

Probabilité

-4      -2      0      2      4

e

Microorganisme n°1515
chevauchement = -0,43

$N_{1515}(m|\bar{\mu}_{1515},\bar{\sigma}_{1515})$

$f_{1515}(m_{1515})$----

$f_{1515}$

$N_{1515}(m|\mu_{1515},\sigma_{1515})$

distance

**Fig. 8**

Probabilité

-4      -2      0      2      4

e

$m_{1515}$= distance mesurée à
la frontière $F_{1515}$

**Fig. 9**

Microorganisme n°325
chevauchement = 1,03

**Fig. 10**

Probabilité

$N_{325}(m|\bar{\mu}_{325},\bar{\sigma}_{325})$

$f_{325}$

$N_{325}(m|\mu_{325},\sigma_{325})$

$f_{325}(m_{325})=0$

distance

$m_{325}= -0,9$

Microorganisme n°59
chevauchement = -0,3

**Fig. 11**

Probabilité

$N_{59}(m|\bar{\mu}_{59},\bar{\sigma}_{59})$

$f_{59}$

$N_{59}(m|\mu_{59},\sigma_{59})$

$f_{59}(m_{59})=0,52$

distance

$m_{59}= -1,1$

**Fig. 12**

Europäisches
Patentamt

European
Patent Office

Office européen
des brevets

# RAPPORT DE RECHERCHE EUROPEENNE

Numéro de la demande

EP 11 30 6609

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | LEE ET AL: "Domain described support vector classifier for multi-classification problems", PATTERN RECOGNITION, ELSEVIER, GB, vol. 40, no. 1, 29 octobre 2006 (2006-10-29), pages 41-51, XP005837154, ISSN: 0031-3203, DOI: 10.1016/J.PATCOG.2006.06.008 * Section 2. "Previous work", Section 3. "The proposed method" * ----- | 1-3,6-9, 14 | INV. G06K9/00 G06K9/62 |
| A | DEBNATH R ET AL: "A DECISION BASED ONE-AGAINST-ONE METHOD FOR MULTI-CLASS SUPPORT VECTOR MACHINE", PATTERN ANALYSIS AND APPLICATIONS, SPRINGER, NEW YORK, NY, US, vol. 7, no. 2, 1 juillet 2004 (2004-07-01), pages 164-175, XP001199266, ISSN: 1433-7541, DOI: 10.1007/S10044-004-0213-6 * le document en entier * ----- | 1-14 | |
| A | LUTS J ET AL: "A tutorial on support vector machine-based methods for classification problems in chemometrics", ANALYTICA CHIMICA ACTA, ELSEVIER, AMSTERDAM, NL, vol. 665, no. 2, 30 avril 2010 (2010-04-30), pages 129-145, XP027019383, ISSN: 0003-2670 [extrait le 2010-04-21] * le document en entier * ----- -/-- | 1-14 | **DOMAINES TECHNIQUES RECHERCHES (IPC)** <br> G06K |

Le présent rapport a été établi pour toutes les revendications

| Lieu de la recherche | Date d'achèvement de la recherche | Examinateur |
|---|---|---|
| La Haye | 12 novembre 2012 | Grigorescu, Cosmin |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

.............................................................................

& : membre de la même famille, document correspondant

EPO FORM 1503 03.82 (P04C02)

Europäisches
Patentamt

European
Patent Office

Office européen
des brevets

# RAPPORT DE RECHERCHE EUROPEENNE

Numéro de la demande

EP 11 30 6609

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | ROHITH K REDDY ET AL: "Chapter 8. Chemometric Methods for Biomedical Raman Spectroscopy and Imaging", EMERGING RAMAN APPLICATIONS AND TECHNIQUES IN BIOMEDICAL AND PHARMACEUTICAL FIELDS BIOLOGICAL AND MEDICAL PHYSICS, BIOMEDICAL ENGINEERING,, 1 janvier 2010 (2010-01-01), pages 179-213, XP008157738, DOI: 10.1007/978-3-642-02649-2_8 * le document en entier * ----- | 1-14 | |
| A | R. Rifkin et al.: "In Defense of One-vs-All Classification", Journal of Machine Learning Research, vol. 5 2004, pages 101-141, XP002686933, Extrait de l'Internet: URL:http://dl.acm.org/citation.cfm?id=1005336 [extrait le 2012-11-05] * le document en entier * ----- | 1-14 | |
| A | SCHMID U ET AL: "Gaussian mixture discriminant analysis for the single-cell differentiation of bacteria using micro-Raman spectroscopy", CHEMOMETRICS AND INTELLIGENT LABORATORY SYSTEMS, ELSEVIER SCIENCE PUBLISHERS B.V. AMSTERDAM, NL, vol. 96, no. 2, 15 avril 2009 (2009-04-15) , pages 159-171, XP026049440, ISSN: 0169-7439, DOI: 10.1016/J.CHEMOLAB.2009.01.008 [extrait le 2009-02-07] * le document en entier * ----- | 1-14 | |

**DOMAINES TECHNIQUES RECHERCHES (IPC)**

Le présent rapport a été établi pour toutes les revendications

| Lieu de la recherche | Date d'achèvement de la recherche | Examinateur |
|---|---|---|
| La Haye | 12 novembre 2012 | Grigorescu, Cosmin |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1253622 A

- EP 1253622 B1

**Littérature non-brevet citée dans la description**

- **JACKSON O. LAY.** Maldi-tof spectrometry of bacteria. *Mass Spectrometry Reviews,* 2001, vol. 20, 172-194

- **R.-E. FAN ; K.-W. CHANG ; C.-J. HSIEH ; X.-R. WANG ; C.-J. LIN.** LIBLINEAR: A Library for Large Linear Classification. *Journal of Machine Learning Research,* 2008, vol. 9, 1871-1874